# EUROPEAN PATENT APPLICATION

(11) **EP 2 783 757 A1**
(43) Date of publication of application: **01.10.2014**
(21) Application number: 12852082.2
(22) Date of filing: 19.11.2012
(51) Int. Cl.: B05B 15/00, A61M 37/00

(54) **OIL-SPRAYING DEVICE**

(30) Priority: 25.11.2011 KR 20110124344
(71) Applicant: Bae, Jeong-Yong, Uiwang-si, Gyeonggi-do 437-765 (KR)
(72) Inventor: Bae, Jeong-Yong, Uiwang-si, Gyeonggi-do 437-765 (KR)
(74) Representative: Chaillot, Geneviève
(86) International application number: PCT/KR2012/009795
(87) International publication number: WO 2013/077607

(57) **Abstract**

An oil spray device is disclosed. According to the present invention, the oil spray device is configured to include a casing unit which has an exit for allowing a user to enter the casing unit, spray units which are mounted on the inner surface of the casing unit to spray oil to skins of the user in the casing unit, and a means for distinguishing between clothes and skins which distinguishes clothes of the user from skins exposed out of the clothes, wherein the oil spray unit uses the means for distinguishing between clothes and skins to distinguish skins of the user from clothes of the user, and then selectively sprays oil to the skins of the user through the spray units.

## Description

### Technical Field

The present invention relates to an oil spray device, more specifically wherein oil may be applied to a user's skins at one time without the need of using his or her hands as well as clothes that the user wears and his or her skins are distinguished from each other so that oil may be selectively applied.

### Background Art

In the summertime, people apply oil that block ultraviolet rays to their skins in order to prevent their skins from getting burned by the sun while enjoying sunbathing on the beaches or swimming in the sea.

And they apply oil to body portions that their hands are unreachable with the help of others when applying oil to their skins. However, when failing to find the help of others, they have difficulties in applying oil to body portions that their hand are unreachable, such as their backs.

In addition, when they apply oil to their skins with their or others' hands, their hands are coated with oil, and thus they have inconvenience in washing hands after applying oil.

As a result, applying oil on the beaches requires a large amount of time as well as causes inconvenience in which people selectively apply oil to skins exposed out of a swimming suit with their hands.

### Disclosure

### Technical Problem

Accordingly, an objective of the present invention is to provide an oil spray device wherein oil may be applied to a user's skins at one time without the need of using his or her hands as well as clothes that the user wears and his or her skins are distinguished from each other so that oil may be selectively applied.

### Technical Solution

The objective of the present invention is achieved by an oil spray device including a casing unit which has an exit for allowing a user to enter the casing unit, spray units which are mounted on the inner surface of the casing unit to spray oil to skins of the user in the casing unit, and a means for distinguishing between clothes and skins which distinguishes clothes of the user from skins exposed out of the clothes, wherein the oil spray unit uses the means for distinguishing between clothes and skins to distinguish skins of the user from clothes of the user, and then selectively sprays oil to the skins of the user through the spray units.

According to one preferred embodiment of the present invention, the means for distinguishing between clothes and skins receives light reflected from the user and analyzes wavelength shifts of the light, and then uses the analyzed wavelength shifts to distinguish clothes from skins.

According to the one preferred embodiment of the present invention, an optical sensor may be configured to include a light receiving unit which receives light reflected from the user, and a calculating unit which analyzes the light transmitted from the light receiving unit to distinguish clothes from skins.

According to the one preferred embodiment of the present invention, the optical sensor may further include a light outputting unit which emits light.

According to the one preferred embodiment of the present invention, the means for distinguishing between clothes and skins may further include a cover which absorbs particular wavelengths of light, so that the user applies oil only to the body portions that are not covered by the cover.

According to the one preferred embodiment of the present invention, the means for distinguishing between clothes and skins may further include a reference color detection sensor which detects a skin color of each user.

### Advantageous Effects

According to the present invention, there is provided an oil spray device wherein oil may be applied to a user's skins at one time without the need of using his or her hands as well as clothes that the user puts on and his or her skins are distinguished from each other so that oil may be selectively and applied.

### Description of Drawings

FIG. 1 illustrates a structure of an oil spray device according to one embodiment of the present invention.
FIGS. 2 and 3 illustrate an operation principle of the oil spray device according to one embodiment of the present invention.
FIG. 4 illustrates an operation principle of an elevator of the oil spray device according to one embodiment of the present invention.
FIG. 5 is a functional block diagram of a structure of the oil spray device according to one embodiment of the present invention.
FIG. 6 illustrates a top of the elevator of the oil spray device according to one embodiment of the present invention.
FIG. 7 illustrates a structure of spray units according another embodiment of the present invention.

### Mode for Invention

Hereinafter, embodiments of the present invention are described in detail with reference to the accompanying drawings. It is to be noted that like reference numerals refer to similar elements throughout the drawings. In addition, redundant and detailed descriptions of known functions and elements that may unnecessarily make the gist of the present invention obscure are omitted.

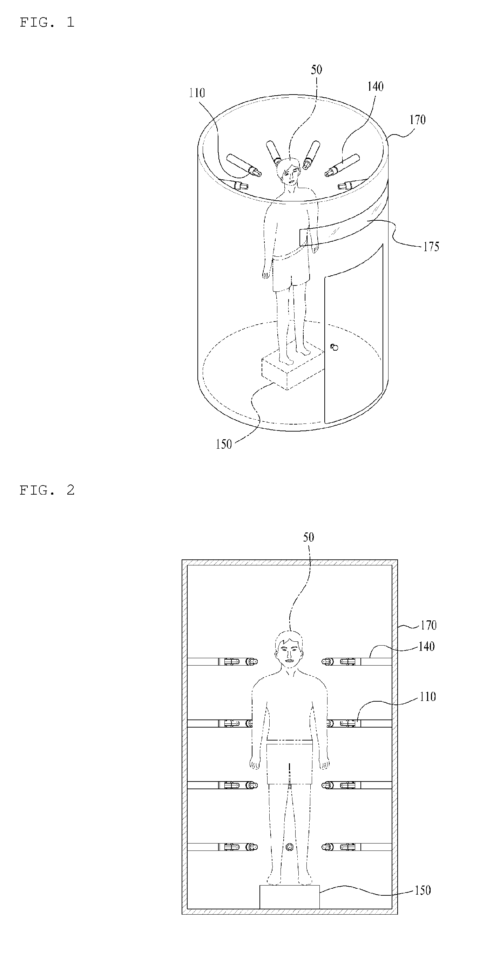

FIG. 1 illustrates a structure of an oil spray device according to one embodiment of the present invention.

Referring to FIG. 1, an oil spray device 100 according to one embodiment of the present invention includes a casing unit 170, spray units 140, and an elevator 150.

As shown in FIG. 1, the casing unit 170 has an exit for allowing a user 50 to enter the casing unit, and also a window 175, which allows the user 50 to watch the outside of the casing unit, formed on the side of the upper portion of the casing unit 170. As described above, the user 50 watches the outside through the window 175 formed on the casing unit 170, thereby reducing the uncomfortable feeling that the user is shut in the casing unit 170.

Furthermore, the spray units 140 are mounted on the inner surface of the casing unit 170, and include oil spray nozzles for spraying oil to skins of the user 50.

To carry out the present invention, oil sprayed from the oil spray nozzles may be selected from the followings: sunblock that blocks ultraviolet rays to prevent skins from being damaged; or sun oil that helps skins to tan regularly and softly.

Besides, to carry out the present invention, the spray units 140 may include air spray nozzles in addition to the oil spray nozzles, wherein the air spray nozzles are used to cool the user in the casing unit 170 and dry oil applied to skins.

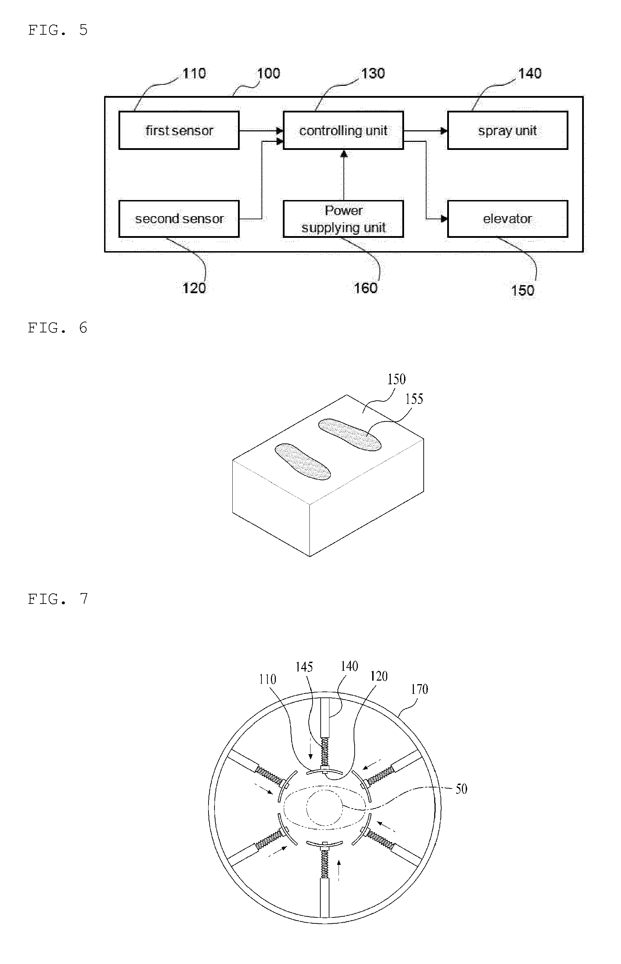

As shown in FIG. 1, the elevator 150 is mounted on the bottom of the casing unit 170, and the user 50 enters the casing unit 170, and then stands on the elevator 150 so that oil is allowed to be applied to his or her skins. Furthermore, to carry out the present invention, it is preferable that foot patterns 155 are provided to a top of the elevator 150 as shown in FIG. 6 so that the user 50 in the casing unit 170 naturally stands on the elevator 150.

Besides, the foot patterns 155 are formed on the top of the elevator 150 in a certain distance, and thus the user naturally stands on the foot patterns 155 in a state in which his or her feet are opened in a certain distance, which prevents the case that oil fails to be evenly applied because his or her thighs come into contact with each other.

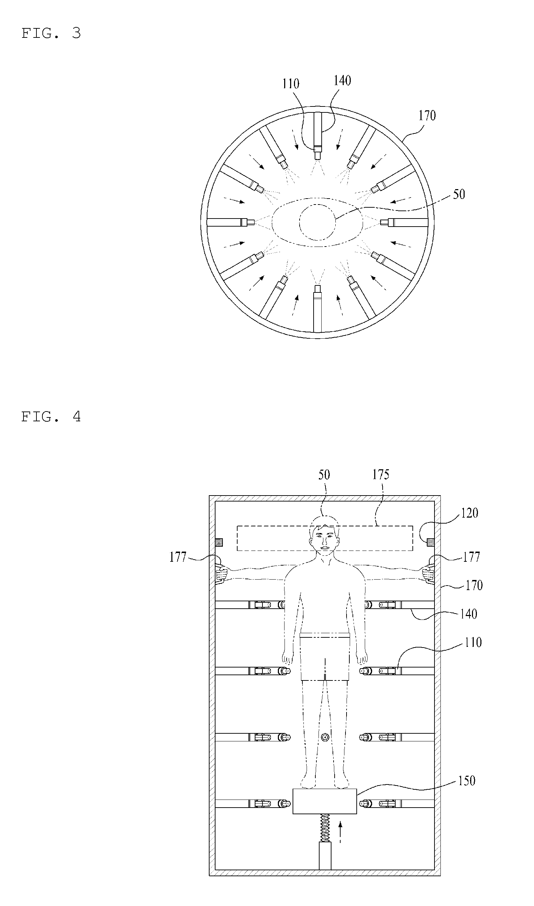

In addition, as shown in FIG. 4, the user grabs handles 177 with both hands when standing on the elevator 150, and thus this prevents the case that oil fails to be applied because both arms block his or her body.

Besides, to carry out the present invention, it is preferable that, as shown in FIGS. 2 and 3, the spray units 140 spray oil to skins of the user 50 after moving closest to skins of the user 50.

In addition, to carry out the present invention, as shown in FIG. 4, it is preferable that, when the user 50 stands on the elevator, the elevator 150 is lifted up until the head of the user 50 reaches the upper portion of the casing unit 170, and more preferable that some nozzles of the spray units 140, that is nozzles disposed below the footboard level of the elevator 150, stop spraying oil, and thus oil is prevented from being wasted.

To carry out this, the oil spray device 100 according to the present invention has a structured as shown in FIG. 5.

FIG. 5 is a functional block diagram of a structure of the oil spray device according to one embodiment of the present invention. Referring to FIG. 5, the oil spray device 100 according the one embodiment of the present invention further includes first sensors 110, second sensors 120, a controlling unit 130, and a power supplying unit 160 in addition to the above-mentioned spray units 140 and elevator 150.

The first sensors 110 are mounted on the circumference of nozzles of the spray units 140 to measure distances between skins of the user 50 and the sensors 110, respectively. Information on the distance measured by the first sensors 110 is transmitted to the controlling unit 130, and then the controlling unit 130 controls the spray units 140 so that the spray units continuously move to the skins until the distances between a plurality of the first sensors 110 and skins of the user 50 are close to a predetermined value (for example, 3cm), respectively, as shown in FIG. 3.

Besides, to carry out the present invention, the first sensors 110 further include contact sensors, and it is preferable that, in a case in which malfunction of the controlling unit 130 causes nozzles of the spray units 140 to come into contact with skins of the user, the controlling unit 130 controls the spray units 140 to stop moving through a signal generated when skins come into contact with the contact sensor.

When distances between the first sensors 110 and skins of the user 50 are equal to a predetermined value in the controlling unit 130, the controlling unit 130 controls the spray units 140 to stop moving and spray oil to skins of the user 50 through spray nozzles of the spray units 140.

Furthermore, the second sensors 120 are mounted in the upper portion of the casing unit 170, and the second sensors 120 determine whether the body portion (head) of the user 50 reaches the upper portion of the casing unit 170.

That is, a pair of the second sensors 120 are mounted in the upper portion of the casing unit 170 in such a manner that the sensors face each other to send and receive infrared rays, wherein a pair of the second sensors 120 receive infrared rays sent to each other when the head of the user 50 does not block a space between a pair of the second sensors 120, and in this case, the controlling unit 130 controls the elevator 150 to be continuously lifted up.

Whereas, when the head of the user 50 blocks a space between a pair of the second sensors 120 after the elevator 150 is lifted up, as shown in FIG. 4, a pair of the second sensors 120 fail to receive infrared rays sent to each other.

As described above, when the second sensors 120 fail to receive infrared rays sent to each other, the controlling unit 130 cuts off electricity supplied to the elevator 150 so that the elevator 150 stops being lifted up, and in this case, the eye level of the user 50 is equal to the window 175 as shown in FIG. 4., and thus the user secures a comfortable eye level.

The oil spray device according to the present invention may further include a means for distinguishing between clothes and skins (not shown) which is used to distinguish clothes of the user from skins exposed out of the clothes. Accordingly, when the user 50 is in the casing unit 170, the means for distinguishing between clothes and skins distinguishes body portions that are covered by a swimming suit (or general clothes) from other body portions that are not covered by the swimming suit, and thus oil is applied only to skins exposed out of the clothes.

A variety of known methods may be applied to the means for distinguishing between clothes and skins. For example, an optical sensor or an image sensor may be used for the means for distinguishing between clothes and skins. The optical or image sensor is conceptually similar to each other, and a device for analyzing wavelengths of received light and determining the shape and size of an object, more specifically determining the shape of a particular portion of the object. Hereinafter, the present invention is described focuses on an optical sensor, wherein the optical sensor conceptually includes an image sensor.

An optical sensor is configured to include a light receiving unit which receives light and a calculating unit which analyzes wavelengths of the received light. The optical analyzes wavelengths of the light received from the light receiving unit to determine body size and body shape of a user and distinguish between clothes and skins of the user. The optical sensor is conceptually similar to human eyes, and objects have colors different from each other, because, when sun rays hit objects, light with particular wavelengths may be absorbed or reflected according to properties and materials of the objects. For example, a black color absorbs all the visible rays, while a white color reflects all the rays. In addition, a sunset occurs because a red color that has the longest wavelength among visible rays and goes a long distance is seen even after the sun sets. As described above, wavelengths of light are different from each other according to objects reflecting the light, and thus the light receiving unit distinguishes clothes from skins exposed out of the clothes when distinguishing a skin color of the user from other colors excluding the skin color. Here, although an error may occur when the user wears clothes having a color same as or similar to his or her skin color, swimming suits are typically made with colors different from skin colors, and thus serious problems are not caused.

Besides, natural light may be used for the light that the light receiving unit receives. Accordingly, an additional light outputting unit is unnecessary when natural light are used. However, it is preferable that a light outputting unit is additionally provided when natural light are used, because measurement environments are variable according to weather conditions. A Light Emitting Diode (LED) lamp may be used for the light outputting unit, or light with particular wavelengths may be used, if necessary. This is variable according to standards of the light receiving unit and calculating unit.

According to the preferred embodiment of the present invention, the oil spray device may further include a reference color detection sensor which detects a skin color of each user. The reference color detection sensor is a means in which the user inputs his or her skin color into the system before using the oil spray device. For example, when the user moves a particular portion of his or her skins or the back of his or her hand close to a skin color detection sensor according to an instruction manual or a user guide before using the oil spray device, the skin color detection sensor begins measuring the skin color of the user. And after that, the optical sensor (image sensor) takes a picture of the user while the user is in the casing unit 170, and then compares a skin color of each body portion in the picture with a reference color to determine that portions having colors different from the reference color are clothes. When the reference color is not provided, a long time may be required or an error may be caused, and this is because a particular color of the light received in the light receiving unit is considered a reference color or a skin color is obtained through a complicated calculation. Accordingly, the above-mentioned problems may be prevented when the reference color detection sensor is provided. Here, the reference color detection sensor may be additionally provided or the light receiving unit may be used instead.

In addition, according to the preferred embodiment of the present invention, the above-mentioned means for distinguishing between clothes and skins may further include a cover for absorbing or reflecting particular wavelengths of light. Accordingly, when portions to which the user does not desire to apply oil (for example, the face or a body portion on which underwear or a brassiere is worn) are covered by the cover, the optical sensor effectively determines portions to be applied with oil.

In general, swimming suits are made using different colors and patterns. Accordingly, when the optical sensor is used to distinguish clothes from body portions, an error may be caused because some colors are similar to a skin color. Although, in most cases, it is not seriously difficult to distinguish clothes from skins because body portions on which a swimming suit is worn are unchangeable and average color changes are used to distinguish clothes from body portions, a complicated calculation process may cause manufacturing costs to rise. Accordingly, using the cover for absorbing or reflecting particular wavelengths of light has an advantage that clothes and skins are easily distinguished from each other through a small amount of calculation. A sensor used for the means for distinguishing between clothes and skins may be provided to different positions in the casing unit 170.

Besides, to carry out the present invention, it is preferable that each of the first sensors 110 mounted on the circumference of a plurality of the spray units 140 has a function for measuring a distance between the sensor and skins using ultrasonic waves as well as further includes a temperature sensor, and thus the first sensors 110 measure temperatures of skins after moving close to the skins.

Specifically, as shown in FIG. 4, in a state in which a plurality of the spray units 140 move close to skins of the user 50, the user 50 desires to apply oil to his or her skins instead of body portions on which a swimming suit is worn.

Accordingly, after moving close to skins of the user 50, the first sensors 110 mounted on the spray unit 140 measure skin temperatures of the user 50, and then transmit the measured temperature values to the controlling unit 130.

Here, some of the first sensors 110 facing skins of the user 50 measure skin temperatures of the user 50, while the other first sensors 110 facing a swimming suit covering skins of the user 50 measure a temperature of the swimming suit having a temperature lower than the skin temperature of the user 50.

The controlling unit 130 that receives the temperature value measured by a plurality of the first sensors 110 does not operate spray nozzles of the spray units 140 that include some of the first sensors 110 measuring a temperature value lower than the other first sensors 110, and thus oil is sprayed only to skins of the user 50 instead of a swimming suit of the user 50.

Alternatively, the controlling unit does not operate the spray nozzle of the spray unit 140 that includes one of the first sensors 110 measuring the lowest temperature value in comparison with the rest of the first sensors 110, because body portions may have temperature values different from each other.

Besides, to carry out the present invention, oil may be sprayed using spray plates 180 which include a plurality of spray nozzles and are made in a form corresponding to body portions, as shown in FIG. 7.

In this case, oil may be sprayed through a plurality of spray nozzles mounted on the spray plates 180 in a state in which the spray plates 180 corresponding to each body portion are moved close to skins by means for moving the spray plates 145 rather than spray nozzles are moved close to skins of the user 50.

Besides, it is preferable that a rotary or crossed method is used for spray nozzles mounted on the spray units 140, and here, the rotary method is not used for the spray nozzles which are disposed at the boundary between clothes and skins.

In addition, a gas spray method used for a sun spray as well as a spray method using air pressure of a compressor such as an air dust gun may be selectively used for the spray nozzles.

Furthermore, to carry out the present invention, another method for rubbing the user's skins with washed and dry gauze may be used instead of spray methods.

Besides, to carry out the present invention, in addition to the above-mentioned method for spraying oil through spray nozzles, another method in which ultraviolet rays blocking agents are manufactured in a gas form and applied to body portions of the user through air may be used, or a variety of spray methods applied to conventional tanning beds may be used.

It is apparent that although the preferred embodiments and examples of the present invention in the foregoing description are illustratively described, the present invention is not limited to the above-mentioned specific embodiments and examples, and various modifications will be made to those skilled in the art without departing from the gist of the present invention defined by the appended claims and these modifications must be understood within the scope of the technical ideas of the present invention.

## Claims

1. Oil spray device, comprising:
a casing unit which has an exit for allowing a user to enter the casing unit;
spray units which are mounted on the inner surface of the casing unit to spray oil to skins of the user in the casing unit; and
a means for distinguishing between clothes and skins which distinguishes clothes of the user from skins exposed out of the clothes,
wherein the oil spray unit uses the means for distinguishing between clothes and skins to distinguish skins of the user from clothes of the user, and then selectively sprays oil to the skins of the user through the spray units.

2. Oil spray device according to claim 1, **characterized in that** the means for distinguishing between clothes and skins is configured with an optical sensor which uses light reflected from the user to distinguish skins from clothes.

3. Oil spray device according to claim 2, **characterized in that** the optical sensor is configured to comprise a light receiving unit which receives light reflected from the user, and a calculating unit which analyzes the light transmitted from the light receiving unit to distinguish clothes from skins.

4. Oil spray device according to claim 3, **characterized in that** the optical sensor further comprises a light outputting unit which emits light.

5. Oil spray device according to claim 4, **characterized in that** the means for distinguishing between clothes and skins further comprises a cover which absorbs particular wavelengths of light, so that the user applies oil only to body portions that are not covered by the cover.

6. Oil spray device according to claim 4, **characterized in that** the means for distinguishing between clothes and skins further comprises a reference color detection sensor which detects a skin color of each user.

7. Oil spray device according to claim 1, **characterized in that** the casing unit further comprises an elevator on which the user stands to apply oil to his or her skins after entering the oil spray device.

8. Oil spray device according to claim 7, **characterized in that** the casing unit further has sensors for detecting the head of the user formed in the upper portion thereof.

9. Oil spray device according to claim 1, **characterized in that** the casing unit has a window, which allows the user to watch the outside thereof, formed on the side of the upper portion thereof.

10. Oil spray device according to claim 1, **characterized in that** the means for distinguishing between clothes and skins uses sensors to measure temperatures of skins exposed out of the user's clothes as well as other temperatures of clothes that the user wears, and the spray units selectively spray oil to the user's skins on the basis of the temperature values measured by the sensors.
